# EUROPÄISCHE PATENTSCHRIFT

(11) **EP 3 062 763 B1**
(45) Veröffentlichungstag und Bekanntmachung des Hinweises auf die Patenterteilung: **20.12.2017**
(21) Anmeldenummer: 14792745.3
(22) Anmeldetag: 30.10.2014
(51) Int. Cl.: A61K 8/34, A61K 8/49, A61K 8/64, A61Q 19/08, A61K 8/97, A61Q 17/00, A61Q 17/04

(54) **VERBESSERTER SCHUTZ VOR EXTRINSISCHER HAUTALTERUNG**
IMPROVED PROTECTION AGAINST EXTRINSIC SKIN AGING
PROTECTION AMÉLIORÉE CONTRE LE VIEILLISSEMENT EXTRINSÈQUE DE LA PEAU

(30) Priorität: 31.10.2013 DE 102013222168
(43) Veröffentlichungstag der Anmeldung: 07.09.2016
(73) Patentinhaber: La Prairie Group AG, 8604 Volketswil (CH)
(72) Erfinder: STANGL, Daniel, CH-6045 Meggen (CH); DUDLER, Bernhard, CH-8340 Hinwil (CH)
(74) Vertreter: Hartmann, Jost
(86) Internationale Anmeldenummer: PCT/EP2014/002916
(87) Internationale Veröffentlichungsnummer: WO 2015/062727

(56) Entgegenhaltungen:
- WO-A1-2012/069073
- WO-A2-2010/086754
- FR-A1- 2 265 401
- FR-A1- 2 273 551
- FR-A1- 2 696 932
- US-A- 5 840 309
- US-A1- 2002 012 644
- "luxurious travel set", GNPD; MINTEL, 31. Dezember 2007 (2007-12-31), XP002673826, [gefunden am 2007-12-01]

## Beschreibung

Die Erfindung ist die Verwendung der Kombination aus a) Glycoprotein 1, b) Glycoprotein 2, c) Ginsengextrakt und d) Schachtelhalmextrakt zum Schutz der epidermalen Stammzellen vor oxidativem Stress und / oder zum Schutz der dermalen Stammzellen vor UV-Licht. Bevorzugt wird die Kombination in Form von kosmetischen oder dermatologischen Zubereitungen bereitgestellt und angewendet. Anspruch 1 betrifft eine Kombination aus (a) Glycoprotein 1, erhältlich aus der gereinigten zytoplasmatischen Fraktion aus Hefen (Saccharomyces), (b) Glycoprotein 2, erhältlich aus der gereinigten zytoplasmatischen Fraktion aus Lactobacillus, (c) Ginsengextrakt und (d) Schachtelhalmextrakt zur Verwendung zum Schutz der epidermalen Stammzellen vor oxidativem Stress und / oder zum Schutz der dermalen Stammzellen vor UV-Licht. Als Hautalterung wird der komplexe biologische Prozess der mit dem Alter einhergehenden Veränderung der Haut bezeichnet. Hierbei unterscheidet man die intrinsische Hautalterung, bedingt durch interne physiologische und genetische Faktoren, von extrinsischer Hautalterung.

Extrinsische Hautalterung ist auf externe Faktoren wie z. B. Umweltfaktoren wie UV-Licht, chemische Reagentien, mechanische Belastung, Zigarettenrauch, Stress oder Luftverschmutzung zurückzuführen. Da UV-Strahlung die Hauptursache für die extrinsische Hautalterung darstellt, spricht man auch von "Photoaging".

Die extrinsischen Faktoren führen beispielsweise zu Faltenbildung, Haut erschlaffen, Verlust von Elastizität und trockenem Aussehen der Haut.

Die intrinsische Hautalterung, auch chronologische Hautalterung genannt, wird durch interne physiologische und genetische Faktoren verursacht und spiegelt Abbauprozesse in der Haut wider. Diese Prozesse sind hauptsächlich auf eine reduzierte Proliferationsaktivität der Hautzellen, eine reduzierte Synthese der Matrixproteine und eine Zunahme der Expression von matrixabbauenden Enzymen zurückzuführen.

Gealterte Zellen zeigen einen Widerstand zu den apoptotischen Signalen, die zur Akkumulation im Gewebe von nicht proliferierenden gealterten Zellen mit verändertem Genexpressionsmuster führt.

Bei der Hautalterung kommt es häufig zur Bildung von Fältchen und Linien und zum Verlust von Elastizität und Spannkraft.

Hautalterung und Faltenbildung können maßgeblich durch entsprechenden Hautschutz verzögert werden. Im Stand der Technik sind hierzu vielerlei Möglichkeiten dargestellt, wie beispielsweise von gesundem Lebenswandel bis hin zu topisch applizierbaren kosmetischen und dermatologischen Zubereitungen.

In der US 5840309 wird die Kombination aus a) Glycoprotein 1, b) Glycoprotein 2, c) Ginsengextrakt und d) Schachtelhalm (horsetail extract) zur Stimulation der Proliferation von Fibroblasten und Keratinozyten beschrieben.

Es ist wünschenswert Zubereitungen zur Verfügung zu stellen, die gegen extrinsische Hautalterung wirksam sind.

Die Erfindung ist die Verwendung der Kombination aus
a) Glycoprotein 1, erhältlich aus der gereinigten zytoplasmatischen Fraktion aus Hefen (Saccharomyces),
b) Glycoprotein 2, erhältlich aus der gereinigten zytoplasmatischen Fraktion aus Lactobacillus,
c) Ginsengextrakt und
d) Schachtelhalmextrakt (horsetail extract, Equisetum Arvense extract) zum Schutz der epidermalen Stammzellen vor oxidativem Stress und / oder zum Schutz der dermalen Stammzellen vor UV-Licht.
Die Kombination aus den Bestandteile a), b), c) und d) wird nachfolgend als Extraktkombination und/oder als GPVE-Extrakt bezeichnet.

Der darin als GP-Extrakt bezeichnete Extrakt umfassend a) kann aus wässrigem Zellextrakt einer gereinigten zytoplasmatischen Fraktion eines natürlichen, ausgewählten Stammes der Hefe Saccharomyces cerevisiae biotechnisch hergestellt werden Der Extrakt enthält Glykoproteine und eine Vielzahl von zellularen Nährstoffen und Faktoren.

Der als VE-Extrakt bezeichnete Extrakt umfassend b), c) und d) und kann durch ein mehrstufiges biotechnologisches Verfahren unter Verwendung von Lactobacillus casei erhalten werden.

Lactobacillus casei, die besonders reich an lytischen Enzymen sind, werden in ein Fermentationskulturmedium überführt, wo sie wachsen können. Dieses Medium enthält auch eiweißreiche grüne Mikroalgen. Unter bestimmten Fermentationsbedingungen lässt man diese Laktobazillen exponentiell wachsen und sie produzieren Enzyme, die in das Medium überführt werden. Diese Enzyme greifen die Zellwände der Algen an und öffnen sie, so dass ihr nährstoffreiches Zytoplasma in das Medium freigesetzt wird.

Während der Fermentationsprozess fortschreitet, werden die Nährstoffe im Medium verbraucht und wenn die Nährstoffe knapp werden, kommt ihr Wachstum zu einem Ende und schließlich zerfallen die Laktobazillen durch Autolyse. Die Zellwände öffnen sich und der Inhalt der Zellen, vor allem Proteine, Enzyme, Vitamine etc. werden in das Medium freigegeben.

In diesem Stadium wird der Prozess gestoppt, das gesamte Medium "geerntet", von Algenresten entfernt und filtriert. Das erhaltene Filtrat stellt das komplette zelluläre Nährstoffsystem dar (CNS), und heißt Lactobacillus Ferment.

Dieses Ferment b) wird mit Extrakten aus Schachtelhalm d) und z.B. Ginseng-Wurzel c) ergänzt, um den endgültigen VE-Extrakt zu bilden.

Glycoprotein 1, ist bspw. im Handel erhältlich z. B. von der Firma DSM, Schweiz, und umfasst eine gereinigte zytoplasmatische Fraktion von Saccharomyces (SACCHAROMYCES CEREVISIAE EXTRACT). Es besteht aus einem Gemisch von Aminosäuren, Nukleinsäuren, Nukleotide, Kohlenhydrate, Lipide, Spurenelementen, Vitaminen und Phosphataseenzyme.

Glykoprotein 2, ist bspw. im Handel erhältlich z. B. von Sederma, Frankreich, und umfasst eine gereinigte zytoplasmatische Fraktion aus Lactobacillus, umfassend eine Mischung aus Aminosäuren, Nukleinsäuren, Nukleotide, Kohlenhydrate, Lipide, Spurenelementen, Vitaminen und Phosphataseenzyme (z.B. LACTOBACILLUS FERMENT).

Der Ginseng-Extrakt ist bspw. erhältlich durch Extraktion mit einem hydrophilen Lösungsmittel (insbesondere Wasser, Ethanol, Glykol, oder beliebige Mischungen daraus) der Wurzel von Panax ginseng. Er enthält Saponine, Sterole, Kohlenhydrate, Pektin, Vitamine, Mineralien und Lipide (z.B. PANAX GINSENG ROOT EXTRACT).

Horsetail Extrakt ist bspw. erhältlich durch Extraktion mit einem hydrophilen Lösungsmittel (z.B. Wasser, Ethanol, Glykol, oder beliebige Mischungen daraus) der ganzen Pflanze von Equisetum arvense. Er enthält Silikate, Flavonoide, Saponoside, Kaffeesäure und Ferulasäure (z.B. EQUISETUM ARVENSE EXTRACT).

Weiterhin erfindungsgemäß ist eine kosmetische oder dermatologische Zubereitung umfassend ein oder mehrere GPVE-Extrakte und ein oder mehrere Verbindungen ausgewählt aus der Gruppe Glycerin, Natriumdehydroacetat, Phenoxyethanol, Kaliumsorbat und/oder Ethylhexylglycerin, bevorzugt alle der genannten Verbindungen.

Der GPVE - Extrakt sowie deren Bestandteile lassen sich in beliebiger Konzentration in kosmetischen oder dermatologischen Zubereitungen einsetzten.

Bevorzugt beträgt der Anteil an einem der GPVE-Extrakten, d.h. ein oder mehreren Extraktkombinationen a.) bis d.), in der Zubereitung bis zu 15 Gew.%, insbesondere bevorzugt bis zu 5 Gew.%, insbesondere im Bereich von 0,0001 bis 0,5 Gew.%, bezogen auf die Gesamtmasse der Zubereitung.

Eine Extraktkombination oder GPVE-Extrakt umfasst dabei immer alle 4 Bestandteile, a.) bis d.). Deren jeweiliger Anteil kann jedoch je nach Ausprägung variiert werden.

Der Anteil an erfindungsgemäßen Extrakten ist definiert als das Verhältnis der Masse des reinen Extraktes, ohne Lösemittel oder Extraktionsmittel, zur Gesamtmasse der Zubereitung.

Die erfindungsgemäßen Zubereitungen können in den bekannten Form und Arten vorliegen. Eine bekannte Form der Zubereitungen sind als leave-on Zubereitungen bekannt, wie Cremes, Lotionen oder Körpermilch. Häufig werden diese als Emulsionen, insbesondere W/O-, O/W-, O/W/O- oder W/O/W-Emulsionen, formuliert. Ebenso können die Zubereitungen Dispersionen, Gele, wässrige oder alkoholische Lösungen, Seren, Öle, Tuchtränkungsmedien, Tinkturen oder Salben darstellen. Die Extrakte lassen sich vorteilhaft auch in Form von oder integriert in Tüchern, Pflaster, Bandagen, Patches oder Pads auf die Haut auftragen.

Gerade der Zusatz an Glycerin, Natriumdehydroacetat, Phenoxyethanol, Kaliumsorbat und/oder Ethylhexylglycerin ermöglicht die Bereitstellung der Kombination a. - d. in einer kosmetischen Zubereitungsform.

Bekannt ist, dass Mitochondrien für die Energieerzeugung in menschlichen Zellen zuständig sind. Sie befinden sich im Zytoplasma und dienen den Zellen als "Batterien", um Energie zu produzieren, zu speichern und zu verteilen. Die menschliche Zelle enthält durchschnittlich 1500 Mitochondrien. Zellen mit hoher Stoffwechselleistung (z.B. Muskeln oder die Leber) enthalten mehr Mitochondrien.

Die Mitochondrien bewegen sich im Zytoplasma je nach Bedarf der Zelle. Sie sind mit Ihrer eigenen DNA ausgestattet und können sich deswegen unabhängig von der Teilung der Zelle eigenständig vermehren. Ohne die Mitochondrien ist die Zelle funktionsunfähig und kein Leben ist möglich.

Wenn diese Kraftwerke der Zellen nicht fehlerfrei arbeiten, kann dies Alterungsprozesse in der Haut beschleunigen. Defekte in den Mitochondrien, insbesondere auch in der mitochondrialen DNA, können daher die Alterung beschleunigen.

Der Schutz der mitochondrialen Funktionalität vor extrinsischen Störfaktoren, z.B. vor UV-Strahlung und oxidativem Stress, bzw. die Gewährleistung der Integrität der Mitochondrien ist daher ein wirksamer Schutz vor der Hautalterung und zählt daher erfindungsgemäß zu einem der wesentlichen Schutzanwendungen vor extrinsischer Hautalterung.

Eine Untersuchung zeigt den Schutz mitochondrialer DNA unter UV-Bestrahlung durch die erfindungsgemäße Kombination.

Auf der mitochondrialen DNA (mtDNA) befinden sich einige, wenn auch nicht alle, Gene für die Enzyme der Atmungskette, sowie Gene, die für die Struktur und Reproduktion der Mitochondrien verantwortlich sind. Schäden können an der mtDNA sehr leicht auftreten, liegt diese doch ungeschützt in den Mitochondrien und ist dort den freien Radikalen, die während der Energieproduktion anfallen können, ausgesetzt. Schäden an der mtDNA können daher zu einer starken Beeinträchtigung der zellulären Energiegewinnung führen.

Einer der häufigsten Schädigungen der mtDNA wird als "common deletion" genannt, die im nachfolgenden Assay detektiert wird.

HaCaT Zellen sind Zellen einer spezifischen humanen Keratinozyten-Zelllinie. Kultivierte Keratinozyten (HaCaT) werden für 48 h mit verschiedenen Verbindungen inkubiert und anschließend mit UVB Strahlung (1,5mJ/cm² für 1 h) gestresst. Die Zellen werden dann gesammelt und lysiert zur DNA-Extraktion. Mit Hilfe der Intensität des common-deletion-Bandes, ausgedrückt als Verhältnis der common deletion gegenüber Standard, kann der Schutz gegen Schädigung durch UV-Licht bestimmt werden.

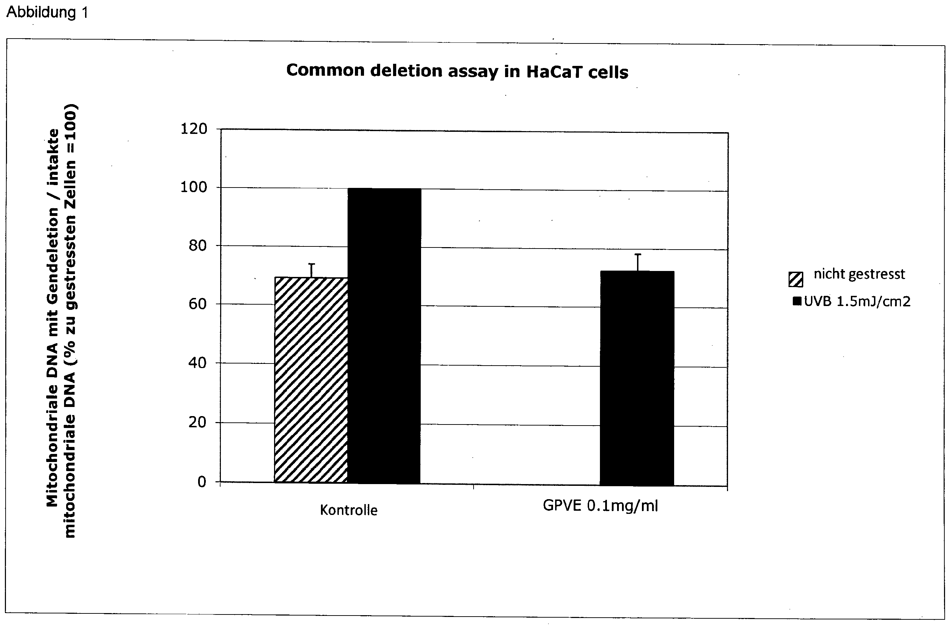

Abbildung 1 zeigt, dass die erfindungsgemäße Kombination, insbesondere in einer kosmetischen Zubereitung mit den optionalen Bestandteilen, die UV-induzierten Schäden der mitochondrialen DNA in Keratinozyten (HaCaT-Zellen) um ca. 90% relativ zur Kontrolle reduziert.

Untersucht wurden 0,1 mg/ml an erfindungsgemäßen GPVE-Extrakten in einer Zubereitung umfassend Glycerin, Natriumdehydroacetat, Phenoxyethanol, Kaliumsorbat und Ethylhexylglycerin.

Es wird ein signifikanter Schutz beobachtet, wie Abbildung 1 zeigt, mit einer 92% Reduktion der UVB induzierten DNA Schäden (Common Deletion Test) gegenüber der Kontrolle.

DNA-Schäden führen zu einer Beeinträchtigung zellulärer Funktionen und letztendlich zu Hautalterung. UV-Strahlung ist der wesentliche Faktor bei vorzeitiger Hautalterung und damit ein extrinsischer Faktor, vor dem die erfindungsgemäße Verwendung schützt.

Epidermale und dermale Stammzellen bzw. deren Nachkommen sind wesentlich am routinemässigen Unterhalt bzw. Erneuerung der entsprechenden Hautschicht beteiligt. Der Schutz dieser Zellen vor externen Stressoren wie UV-Strahlung oder freien Radikalen trägt somit wesentlich zum Erhalt einer jugendlichen, gesunden und schöner Haut bei.

Die erfindungsgemäße Zubereitung mit GPVE-Extrakt, bspw. zu einem Anteil an 0,125 Gew.%, bezogen auf die Gesamtmasse der Zubereitung, schützt das Proliferationspotenzial epidermaler Stammzellen unter oxidativem Stress. Gemessen wird dies an der Fähigkeit der Zellen, Kolonien in-vitro zu bilden (Colony Forming Efficiency = CFE).
Dazu wurden folgende Experimente durchgeführt.

Primäre humane epidermalen Keratinozyten Vorläuferzellen werden in Gegenwart der Zubereitung mit GPVE-Extrakt in CnT-07 Kulturmedien ausgesät und man lässt sie für 48 Stunden wachsen. Die Testmuster werden dann verschiedenen Konzentrationen von Wasserstoffperoxid ausgesetzt. Danach werden die Zellen für das CFE Assays ausgesät. Für die CFE Auswertung werden die Zellen für 10 Tage mit einer niedrigen Dichte ausgesät und gezüchtet. Die Kulturen werden dann fixiert, gefärbt und die Kolonien gezählt. Die CFE Auswertungen werden in dreifacher Ausführung durchgeführt. Unbehandelte Zellen werden als Kontrolle verwendet.

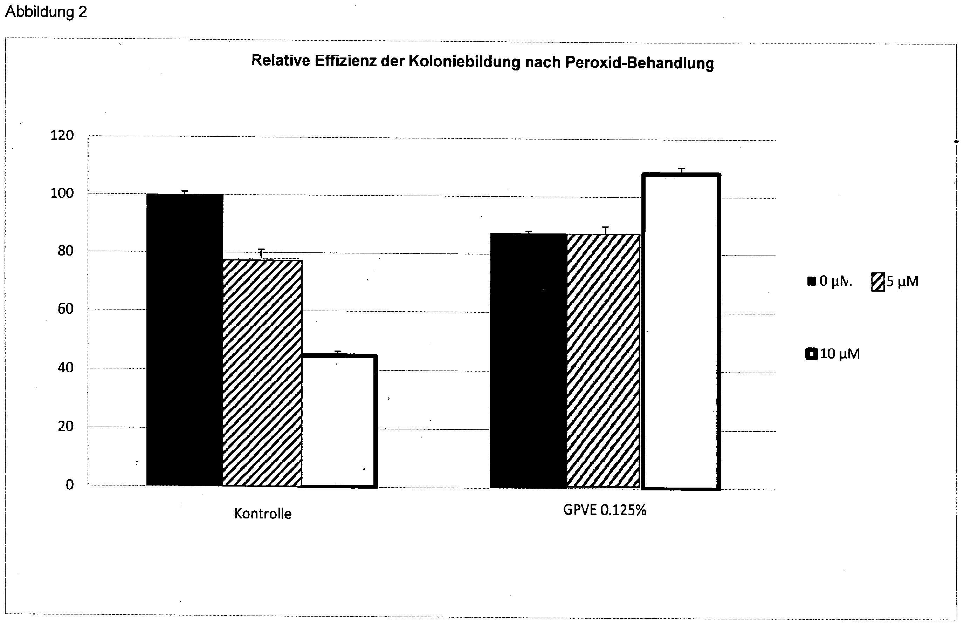

Wie die Resultate in der Abbildung 2 zeigen, schützt die erfindungsgemäße Zubereitung umfassend GPVE-Extrakte das Proliferationspotential der epidermalen Stammzellen signifikant vor oxidativem Stress durch Wasserstoffperoxid. So erhöht sich die Anzahl der gebildeten Kolonien in Gegenwart des Extraktes um mehr als das Doppelte im Vergleich zur unbehandelten Kontrolle bei 10µM Wasserstoffperoxid.

Der erfindungsgemäß bevorzugte GPVE-Extrakt bzw. die ihn enthaltenen Zubereitungen schützen das Proliferationspotenzial epidermaler Stammzellen unter UV- und oxidativem Stress, gemessen an der Fähigkeit der Zellen, Kolonien in-vitro zu bilden (Colony Forming Efficiency = CFE). Dazu wurden folgende Experimente durchgeführt:

### UV Bestrahlung:

Mit zwei gewählten Konzentrationen werden die primären epidermalen Keratinozyten Vorläuferzellen in Gegenwart von jeder Verbindung in CnT-07 Kulturmedium ausgesät und man lässt sie für 48 Stunden wachsen. Jeder Test wird dann einer Bestrahlung mit einer UVA-und UVB-Lichtquelle entweder 1200 mJ oder 1800 mJ ausgesetzt. Ein Test mit nicht bestrahlter Probe wird auch durchgeführt. Nach der Belichtung werden die Zellen für das CFE Assays ausgesät. Für die CFE Auswertung werden die Zellen für 10 Tage mit einer niedrigen Dichte ausgesät und gezüchtet. Die Kulturen werden dann fixiert, gefärbt und die Kolonien gezählt. Die CFE Auswertungen werden in dreifacher Ausführung durchgeführt. Unbehandelte Zellen werden als Kontrolle verwendet.

Der GPVE-Extrakt schützt dermale Stammzellen gegen die negativen Auswirkungen von UV-Strahlung auf deren Proliferationsfähigkeit. Auch damit zeigt sich die überraschende Verwendungsmöglichkeit der GPVE Extrakte zum Schutz der Haut vor extrinsischer Hautalterung.

Folgendes Experiment wurde dazu durchgeführt: Dermale Vorläuferzellen (isoliert aus dermalen Papille) werden in Monoschichten in Gegenwart oder Abwesenheit der Extrakte für einen Zeitraum von 2 Tage kultiviert, dann mit einer UVA-und UVB-Lichtquelle mit einer Dosis von 1200 mJ oder 1800 mJ bestrahlt.

Eine Sphärenbildung wird nach ca. 5 Tage der Kultur ausgewertet.

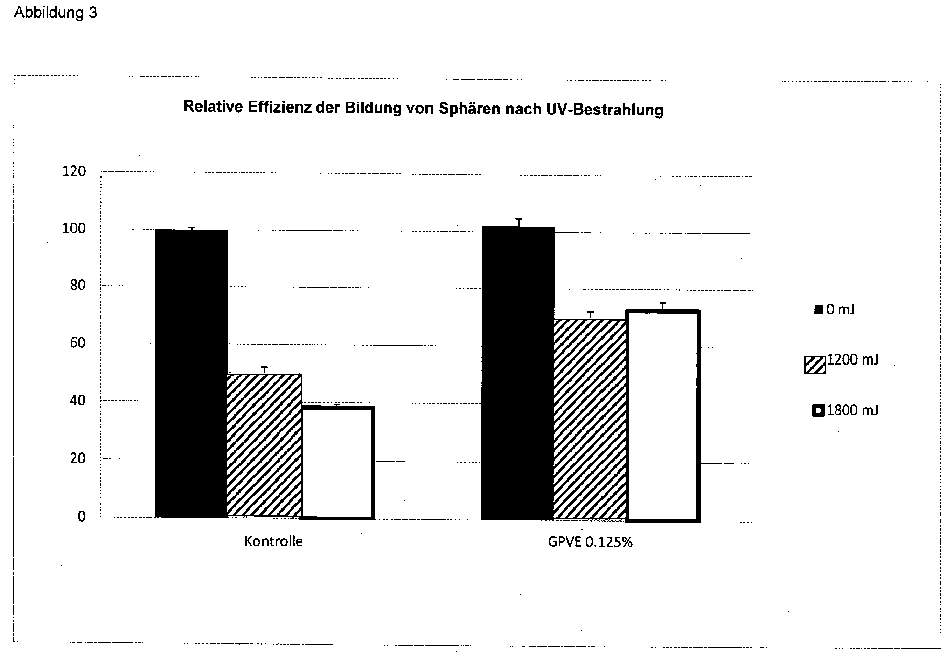

Die Proliferationsfähigkeit der dermalen Stammzellen wird anhand ihrer Fähigkeit, sphärische Kolonien zu bilden (Sphären) ermittelt. Sinkt in der Kontrolle (ohne Extrakte) die Anzahl der gebildeten Sphären um 50% bzw. 62% unter UV-Bestrahlung (1200 mJ bzw. 1800 mJ), so steigt die Anzahl der Sphären in Gegenwart von GPVE um 40% bzw. 92% (Abbildung 3).

Die erfindungsgemäßen Zubereitungen mit GPVE-Extrakten haben einen stimulierenden Effekt auf die Synthese wichtiger Hautlipide in der Epidermis. Diese Lipide, insbesondere Ceramide, sind wesentlich für den Aufbau einer intakten Hautbarriere. Folgendes Experiment wurde dazu durchgeführt.

Humane epidermale Keratinozyten wurden in einem Kulturmedium ausgesät, für 24 Stunden kultiviert, nach weiteren 7 Tagen Inkubation im Medium in Gegenwart von radioaktiv markiertem Acetat, mit und ohne GPVE-Extrakt und mit Calciumchlorid/Vitamin C als positiver Kontrolle, gewaschen und lysiert. Die so erhaltenen Zellextrakte wurden mittels Dünnschichtchromatografie analysiert und die einzelnen Spots über ihre Radioaktivität quantifiziert.

### Resultat:

Der GPVE-Extrakt stimuliert signifikant die Synthese der wichtigsten Klassen von Hautlipiden wie folgt:

| Lipidklasse | % GPVE | Stimulation (% Kontrolle) |
|---|---|---|
| Sphingomyeline | 0.0007 | 121 |
| Phospholipide | 0.0062 | 137 |
| Cholesterol Sulfat | 0.002 | 126 |
| Polare Ceramide und Cerebroside | 0.002 | 131 |
| Wenig polare Ceramide und Cerebroside | 0.0007 | 160 |

Der Extrakt stimuliert unerwarteterweise die Synthese von Sphingomyelin, Phospholipiden, Cholesterolsulfat, Ceramide and Cerebroside in humanen Keratinozyten. Der Anteil an GPVE-Extrakten in der Anwendungszubereitung beträgt dabei nur 0,0007 bis 0,002 Gew.%.

Die erfindungsgemäßen Zubereitungen und insbesondere der GPVE Extrakt kann daher bevorzugt zu folgenden Schutz bzw. Stimulation verwendet werden:
1. Schutz der epidermalen Stammzellen vor oxidativem Stress
2. Schutz der dermalen Stammzellen vor UV-Licht
3. Stimulation der Lipidsynthese

Alle drei Effekte waren nicht zu erwarten.

Die dargestellten Versuche belegen die Anwendungsmöglichkeit der Extraktkombination (GPVE) aus
a) Glycoprotein 1, erhältlich aus der gereinigten zytoplasmatischen Fraktion aus Hefen (Saccharomyces),
b) Glycoprotein 2, erhältlich aus der gereinigten zytoplasmatischen Fraktion aus Lactobacillus,
c) Ginsengextrakt und
d) Schachtelhalmextrakt (horsetail extract, Equisetum Arvense extract)
sowie ggf. zusätzlich Glycerin, Natriumdehydroacetat, Phenoxyethanol, Kaliumsorbat und Ethylhexylglycerin, vorteilhaft in Form kosmetischer oder dermatologischer Zubereitungen, als Schutz vor Hautschäden, die durch extrinsische Faktoren auftreten können.

Die kosmetischen oder dermatologischen Zubereitungen gemäß der Erfindung können ferner Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der Schutzfunktion und Stimulation nicht beeinträchtigt.

Die erfindungsgemäße Zubereitung umfassend ein oder mehrere GPVE-Extrakte umfasst bevorzugt ein oder mehrere Verbindungen ausgewählt aus der Gruppe Glycerin, Natriumdehydroacetat, Phenoxyethanol, Kaliumsorbat und/oder Ethylhexylglycerin, bevorzugt alle der genannten Verbindungen. Diese Zubereitungen erweisen sich überraschend als besonders stabil und hautverträglich, so dass die geschilderten Schutzfunktionen und Stimulationen auch in der täglichen Anwendung erfolgen können.

Zudem zeigte sich, dass diese Bestandteile die Schutzfunktion der GPVE Extrakte weiter steigern helfen.

Erfindungsgemäß ist daher eine kosmetische oder dermatologische Zubereitung umfassend ein oder mehrere GPVE-Extrakte und ein oder mehrere Verbindungen ausgewählt aus der Gruppe Glycerin, Natriumdehydroacetat, Phenoxyethanol, Kaliumsorbat und/oder Ethylhexylglycerin, bevorzugt alle der genannten Verbindungen.

Ein oder mehrere GPVE-Extrakte können zur Verminderung oder Vermeidung von Hautschäden durch extrinsische Faktoren verwendet werden.

Ein oder mehrere GPVE-Extrakte können zur Herstellung pharmazeutischer, insbesondere dermatologischen Zubereitungen verwendet werden und zur Verminderung oder Vermeidung von Hautschäden durch extrinsische Faktoren dienen.

Die erfindungsgemäßen Extrakte sind bevorzugt in topisch applizierbaren Zubereitungen enthalten.

Insbesondere ist die topisch applizierbare Zubereitung eine kosmetische Zubereitung.

Bei Einschränkungen auf bevorzugt genannte Stoffe, seien es die Extrakte, Lipide oder Wirkstoffe oder weitere bevorzugt gennannte Bestandteile, so beziehen sich deren bevorzugten Anteilsbereiche dann auch auf die dann ausgewählten Einzelbestandteile. Die anderen, die durch die Einschränkung ausgeschlossenen Bestandteile, zählen dann nicht mehr zu den aufgeführten Anteilsbereichen hinzu

Nachfolgende Beispiele erläutern die erfindungsgemäßen Zubereitungen.

### Beispiel 1 - O/W-Emulsion mit SPF

Diese Zubereitung kann vorteilhaft noch folgende Komponenten enthalten: Peptide, Pflanzenextrakte, Extrakte pflanzlicher Stammzellen, Biopolymere, Vitamine, Antioxidantien.

| INCI-Bezeichnung | Gew. % |
|---|---|
| WATER (AQUA) | 53,7277 |
| ETHYLHEXYL METHOXYCINNAMATE | 7,5000 |
| ETHYLHEXYL SALICYLATE | 5,0000 |
| C12-15 ALKYL ETHYLHEXANOATE | 5,0000 |
| GLYCERIN | 3,5238 |
| DIMETHICONE/VINYL DIMETHICONE CROSSPOLYMER | 3,2125 |
| BUTYL METHOXYDIBENZOYLMETHANE | 3,0000 |
| POLYGLYCERYL-3 METHYLGLUCOSE DISTEARATE | 2,5500 |
| OCTOCRYLENE | 2,5000 |
| HELIANTHUS ANNUUS (SUNFLOWER) SEED OIL UNSAPONIFIABLES | 2,0000 |
| BEHENYL ALCOHOL | 1,5000 |
| STEARETH-21 | 1,5000 |
| BUTYLENE GLYCOL | 1,4100 |
| CAPRYLIC/CAPRIC TRIGLYCERIDE | 0,9650 |
| HYDROGENATED LECITHIN | 0,5040 |
| TOCOPHERYL ACETATE | 0,5000 |
| CETYL DIMETHICONE | 0,5000 |
| CAPRYLYL GLYCOL | 0,4200 |
| STEARETH-2 | 0,4000 |
| PEG-100 STEARATE | 0,3750 |
| GLYCERYL STEARATE | 0,3750 |
| POTATO STARCH MODIFIED | 0,3000 |
| POLYACRYLAMIDE | 0,2625 |
| CARNOSINE | 0,2500 |
| PANTHENOL | 0,2500 |
| DISODIUM EDTA | 0,2080 |
| SODIUM PCA | 0,2000 |
| CARBOMER | 0,2000 |
| UREA | 0,2000 |
| C13-14 ISOPARAFFIN | 0,1275 |
| ALCOHOL | 0,1105 |
| ALLANTOIN | 0,1000 |
| LAURETH-7 | 0,0600 |
| YNTHETIC WAX | 0,0460 |
| SILICA | 0,0375 |
| SODIUM HYDROXIDE | 0,0330 |
| POLYQUATERNIUM-51 | 0,0252 |
| SODIUM HYALURONATE | 0,0180 |
| TRIACETIN | 0,0080 |
| GLYCINE SOJA (SOYBEAN) OIL | 0,0080 |
| HYDROXYETHYL BEHENAMIDOPROPYL DIMONIUM CHLORIDE | 0,0070 |
| POLYSORBATE 80 | 0,0060 |
| LACTOBACILLUS FERMENT | 0,0052 |
| DEXTRAN | 0,0018 |
| PANAX GINSENG ROOT EXTRACT | 0,0018 |
| SACCHAROMYCES CEREVISIAE EXTRACT | 0,0018 |
| POLYQUATERNIUM-67 | 0,0010 |
| EQUISETUM ARVENSE EXTRACT | 0,0009 |
| ETHYLHEXYLGLYCERIN | 0,0005 |
| SODIUM OLEATE | 0,0004 |
| GLYCOPROTEINS | 0,0003 |
| FRAGRANCE (PARFUM) | 0,4333 |
| PHENOXYETHANOL | 0,5621 |
| SORBIC ACID | 0,0625 |
| CHLORPHENESIN | 0,0030 |
| METHYLPARABEN | 0,0014 |
| BENZOIC ACID | 0,0012 |
| SODIUM DEHYDROACETATE | 0,0011 |
| POTASSIUM SORBATE | 0,0007 |
| YELLOW 5 | 0,0005 |
| RED 4 | 0,0003 |
| | 100,0000 |

### Beispiel 2 - Serum

Diese Zubereitung kann vorteilhaft noch folgende Komponenten enthalten: Peptide, Pflanzenextrakte, Extrakte pflanzlicher Stammzellen, Biopolymere, Vitamine, Antioxidantien.

| INCI-Bezeichnung | Gew. % |
|---|---|
| WATER (AQUA) | 74,6950 |
| PROPYLENE GLYCOL | 5,6991 |
| SD ALCOHOL 40-B (ALCOHOL DENAT.) | 5,3001 |
| BIS-PEG-18 METHYL ETHER DIMETHYL SILANE | 4,3000 |
| GLYCERIN | 3,1381 |
| PENTYLENE GLYCOL | 1,6500 |
| SEA WATER (MARIS AQUA) | 0,9800 |
| ETHYLHEXYL PALMITATE | 0,9530 |
| PANTHENOL | 0,6000 |
| ACRYLATES/C10-30 ALKYL ACRYLATE CROSSPOLYMER | 0,3000 |
| XANTHAN GUM | 0,2500 |
| HDI/TRIMETHYLOL HEXYLLACTONE CROSSPOLYMER | 0,1960 |
| ISOMALT | 0,1850 |
| BUTYLENE GLYCOL | 0,1600 |
| DISODIUM EDTA | 0,1040 |
| ETHYLHEXYL METHOXYCINNAMATE | 0,1000 |
| HYDROXYETHYLCELLULOSE | 0,1000 |
| BUTYL METHOXYDIBENZOYLMETHANE | 0,0960 |
| PPG-26-BUTETH-26 | 0,0920 |
| SODIUM HYDROXIDE | 0,0783 |
| ETHYLHEXYL SALICYLATE | 0,0600 |
| SODIUM HYALURONATE | 0,0520 |
| PEG-40 HYDROGENATED CASTOR OIL | 0,0520 |
| ETHYLHEXYLGLYCERIN | 0,0500 |
| DISODIUM ADENOSINE TRIPHOSPHATE | 0,0300 |
| SILICA DIMETHYL SILYLATE | 0,0250 |
| LECITHIN | 0,0210 |
| ALCOHOL | 0,0200 |
| CAPRYLYL GLYCOL | 0,0060 |
| ALGIN | 0,0055 |
| SILICA | 0,0040 |
| GLYCINE SOJA (SOYBEAN) OIL | 0,0020 |
| HYDROGENATED LECITHIN | 0,0020 |
| SODIUM CHLORIDE | 0,0018 |
| LACTOBACILLUS FERMENT | 0,0017 |
| SODIUM PHOSPHATE | 0,0014 |
| HEXYLENE GLYCOL | 0,0010 |
| EDTA | 0,0010 |
| POLYSORBATE 80 | 0,0010 |
| SACCHAROMYCES CEREVISIAE EXTRACT | 0,0006 |
| PANAX GINSENG ROOT EXTRACT | 0,0006 |
| EQUISETUM ARVENSE EXTRACT | 0,0003 |
| SODIUM OLEATE | 0,0002 |
| GLYCOPROTEINS | 0,0001 |
| FRAGRANCE (PARFUM) | 0,2030 |
| PHENOXYETHANOL | 0,4774 |
| POTASSIUM SORBATE | 0,0019 |
| SODIUM BENZOATE | 0,0007 |
| SODIUM DEHYDROACETATE | 0,0004 |
| EXT. VIOLET 2 | 0,0007 |
| BLUE 1 | 0,0001 |
| | 100,0000 |

## Patentansprüche

1. Kombination aus
a) Glycoprotein 1, erhältlich aus der gereinigten zytoplasmatischen Fraktion aus Hefen (Saccharomyces),
b) Glycoprotein 2, erhältlich aus der gereinigten zytoplasmatischen Fraktion aus Lactobacillus,
c) Ginsengextrakt und
d) Schachtelhalmextrakt (horsetail extract, Equisetum Arvense extract)
zur Verwendung zum Schutz der epidermalen Stammzellen vor oxidativem Stress und/oder zum Schutz der dermalen Stammzellen vor UV-Licht.

2. Kombination zur Verwendung nach Anspruch 1 in einer kosmetischen Zubereitung.

3. Kombination zur Verwendung nach Anspruch 2 **dadurch gekennzeichnet, dass** der oder die Extraktkombination a.) bis d.) zu einem Anteil von bis zu 5 Gew.%, insbesondere im Bereich von 0,0001 bis 0,5 Gew.%, bezogen auf die Gesamtmasse der Zubereitung, in der Zubereitung enthalten sind.

4. Kombination zur Verwendung nach Anspruch 2 oder 3 umfassend ein oder mehrere Verbindungen ausgewählt aus der Gruppe Glycerin, Natriumdehydroacetat, Phenoxyethanol, Kaliumsorbat und/oder Ethylhexylglycerin.

## Claims

1. Combination of
a) glycoprotein 1 obtainable from the purified cytoplasmatic fraction from yeasts (Saccharomyces),
b) glycoprotein 2 obtainable from the purified cytoplasmatic fraction from Lactobacillus,
c) ginseng extract and
d) horsetail extract (Equisetum Arvense extract) for use and/or for protecting the epidermal stem cells against oxidative stress for protecting the dermal stem cells against UV light.

2. Combination for use according to Claim 1 in a cosmetic preparation.

3. Combination for use according to Claim 2, **characterized in that** the or the extract combination a.) to d.) are present in the preparation in a fraction of up to 5% by weight, in particular in the range from 0.0001 to 0.5% by weight, based on the total mass of the preparation.

4. Combination for use according to Claim 2 or 3, comprising
one or more compounds selected from the group glycerol, sodium dehydroacetate, phenoxyethanol, potassium sorbate and/or ethylhexylglycerol.

## Revendications

1. Combinaison
a) d'une glycoprotéine 1, pouvant être obtenue à partir de la fraction cytoplasmique purifiée de levures (Saccharomyces),
b) d'une glycoprotéine 2, pouvant être obtenue à partir de la fraction cytoplasmique purifiée de Lactobacillus,
c) d'extrait de ginseng et
d) d'extrait de prêle ("horsetail extract", "Equisetum Arvense extract")
pour une utilisation pour la protection des cellules souches épidermiques contre le stress oxydatif et/ou pour la protection des cellules souches dermiques contre la lumière UV.

2. Combinaison pour une utilisation selon la revendication 1 dans une préparation cosmétique.

3. Combinaison pour une utilisation selon la revendication 2, **caractérisée en ce que** le ou la combinaison d'extraits a) à d) sont contenue dans la préparation en une proportion allant jusqu'à 5% en poids, en particulier dans la plage de 0,0001 à 0,5% en poids, par rapport à la masse totale de la préparation.

4. Combinaison pour une utilisation selon la revendication 2 ou 3, comprenant un ou plusieurs composés, choisis dans le groupe formé par le glycérol, le déshydroacétate de sodium, le phénoxyéthanol, le sorbate de potassium et/ou l'éthylhexylglycérol.
